# EUROPEAN PATENT SPECIFICATION

(11) **EP 4 059 425 B1**
(45) Date of publication and mention of the grant of the patent: **27.12.2023**
(21) Application number: 22161141.1
(22) Date of filing: 09.03.2022
(51) Int. Cl.: A61B 5/145, A61B 5/361, A61B 5/00

(54) **DETECTION AND/OR PREDICTION OF A MEDICAL CONDITION USING ATRIAL FIBRILLATION AND GLUCOSE MEASUREMENTS**
DETEKTION UND/ODER VORHERSAGE EINES MEDIZINISCHEN ZUSTANDS UNTER VERWENDUNG VON VORHOFFLIMMER- UND GLUKOSEMESSUNGEN
DÉTECTION ET/OU PRÉDICTION D'UNE CONDITION MÉDICALE À L'AIDE DE LA FIBRILLATION AURICULAIRE ET DE MESURES DU GLUCOSE

(30) Priority: 17.03.2021 US 202117204573
(43) Date of publication of application: 21.09.2022
(73) Proprietor: Covidien LP, Mansfield, MA 02048 (US)
(72) Inventor: Kinzie, Patrick, Glendale, AZ 85281 (US); Probst, David, Chandler, AZ 85281 (US); Askarinya, Mohsen, Chandler, AZ 85281 (US); Gilletti, Aaron, Mansfield, MA 02048 (US); O'Brien, Richard, Minneapolis, MN 55432 (US); Phelps, Mark, Tempe, AZ 85281 (US); Schulhauser, Randal, Tempe, AZ 85281 (US); Wainwright, John, Irvine, CA 92617 (US)
(74) Representative: Zimmermann & Partner Patentanwälte mbB

(56) References cited:
- KR-A- 20210 026 711
- US-A1- 2020 187 865
- US-B1- 8 708 906
- TAKASE KEI-ICHIRO ED - LUEF GERHARD ET AL: "Cardiogenic cerebral infarction in the parietal lobe predicts the development of post-stroke epilepsy", SEIZURE, BAILLIERE TINDALL, LONDON, GB, vol. 80, 20 June 2020 (2020-06-20), pages 196-200, XP086246237, ISSN: 1059-1311, DOI: 10.1016/J.SEIZURE.2020.06.018 [retrieved on 2020-06-20]

## Description

### TECHNICAL FIELD

This disclosure is directed to medical devices and, more particularly, to systems and methods for detecting and/or predicting medical conditions, such as stroke.

### BACKGROUND

Stroke is a serious medical condition that can cause permanent neurological damage, complications, and death. Stroke may be characterized as the rapidly developing loss of brain functions due to a disturbance in the blood vessels supplying blood to the brain. The loss of brain functions can be a result of ischemia (lack of blood supply) caused by thrombosis, embolism, or hemorrhage. The decrease in blood supply can lead to dysfunction of the brain tissue in that area.

Stroke is the number two cause of death worldwide and the number one cause of disability. Speed to treatment is the critical factor in stroke treatment as 1.9M neurons are lost per minute on average during a stroke. Stroke diagnosis and time between event and therapy delivery are the primary barriers to improving therapy effectiveness. Stroke has three primary etiologies: i) ischemic stroke (representing about 65% of all strokes), ii) hemorrhagic stroke (representing about 10% of all strokes), and iii) cryptogenic strokes (representing 25% of all strokes, and including transient ischemic attack, or TIA). Strokes can be considered as having neurogenic and/or cardiogenic origins.

A variety of approaches exist for treating patients undergoing a stroke. For example, a clinician may administer anticoagulants, such as warfarin, or may undertake intravascular interventions such as thrombectomy procedures to treat ischemic stroke. As another example, a clinician may administer antihypertensive drugs, such as beta blockers (e.g., Labetalol) and ACE-inhibitors (e.g., Enalapril) or may undertake intravascular interventions such as coil embolization to treat hemorrhagic stroke. Lastly, if stroke symptoms have been resolved on their own with negative neurological work-up, a clinician may administer long-term cardiac monitoring (external or implantable) to determine potential cardiac origins of cryptogenic stroke. US 2020/0187865 A1 describes a system for computing heart failure risk.

### SUMMARY

In general, the disclosure is directed to devices, systems, and techniques for detecting and/or predicting medical conditions, such as stroke, based on detected atrial fibrillation and glucose levels. Atrial fibrillation and glucose levels may be detected via one or more medical devices, e.g., implantable medical devices (IMDs) and/or external medical devices. In some examples, the techniques may include determining respective first and second metrics based on atrial fibrillation glucose levels detected during a time unit, and generating a health metric based on the first and second metrics. The techniques may include outputting an alert based on the health metric, e.g., based on a comparison of the health metric to one or more thresholds. In some examples, the health metric may indicate or be used to determine a probability that the patient is experiencing or will experience a stroke. In some examples, the health metric may be determined based on one or more additional parameters of the patient, such as bioimpedance, hematocrit level, or one or more other parameters that indicate a hydration level or volume of circulating blood of the patient.

In one example, a system includes electrocardiogram sensing circuitry configured to sense an electrocardiogram of a patient; glucose sensing circuitry configured to sense glucose levels of the patient; and processing circuitry configured to: detect atrial fibrillation of the patient during a time unit based on the electrocardiogram of the patient, determine a first metric, wherein the first metric is associated with atrial fibrillation the patient experiences during the time unit, determine a second metric, wherein the second metric is associated with glucose levels of the patient during the time unit, and generate a health metric, wherein the health metric is determined based on the first and second metrics.

In another example, a method includes sensing an electrocardiogram of a patient; sensing glucose levels of the patient; detecting atrial fibrillation of the patient during a time unit based on the electrocardiogram of the patient; determining a first metric, wherein the first metric is associated with atrial fibrillation the patient experiences during the time unit; determining a second metric, wherein the second metric is associated with glucose levels of the patient during the time unit; and generating a health metric, wherein the health metric is determined based on the first and second metrics.

In another example, a computer readable storage medium includes instructions that, when executed, cause processing circuitry to perform any of the methods described herein.

The current invention is defined by the appended claims.

### BRIEF DESCRIPTION OF THE DRAWINGS

FIG. 1A is a conceptual diagram of an example medical system in conjunction with a patient.
FIG. 1B is a conceptual diagram of another example medical system in conjunction with a patient.
FIG. 1C is a diagram of the 10-20 map for electroencephalography (EEG) sensor measurements.
FIG. 1D is a conceptual diagram of another example medical system in conjunction with a patient.
FIG. 1E is a conceptual diagram of another example medical system in conjunction with a patient.
FIG. 2 is a functional block diagram illustrating an example configuration of a medical device in accordance with examples of the present disclosure.
FIG. 3 is a conceptual side-view diagram illustrating an example medical device of a medical system in accordance with examples of the present disclosure.
FIG. 4 is a functional block diagram illustrating an example configuration of an external computing device in accordance with examples of the present disclosure.
FIG. 5 is a block diagram illustrating an example system that includes an access point, a network, external computing devices, such as a server, and one or more other computing devices, which may be coupled to the medical device(s) and external computing devices of FIGS. 1A to 4.
FIG. 6 is a flow diagram illustrating an example operation for generating a health metric indicative of the risk of stroke of a patient based on signals sensed via one or more medical devices, in accordance with one or more techniques disclosed herein.
FIG. 7 is a flow diagram illustrating an example operation for generating an alert based on a health metric determined by one or more medical devices, in accordance with one or more techniques disclosed herein.
FIG. 8 is a flow diagram illustrating an example operation for generating a health metric trend for display, in accordance with one or more techniques of this disclosure.
FIG. 9A is a graph illustrating an example historical health metric trend generated according to the techniques of this disclosure.
FIG. 9B is a graph illustrating another example historical health metric trend generated according to the techniques of this disclosure.

Like reference characters denote like elements throughout the description and figures.

### DETAILED DESCRIPTION

Medical devices can sense and monitor signals and use those signals to determine various conditions of a patient and/or provide therapy to the patient. Example medical devices include implantable monitors, such as the Reveal LINQ^{™} Insertable Cardiac Monitor, available from Medtronic, PLC, of Dublin, Ireland. Another example medical device is the Guardian^{™} Sensor 3, available from Medtronic, PLC, of Dublin, Ireland, for continuous glucose monitoring. Such medical devices may facilitate relatively longer-term monitoring of patients during normal daily activities, and may periodically transmit collected data to a network service, such as the Medtronic CareLink^{®} Network, developed by Medtronic, PLC, or some other network linking a patient to a clinician.

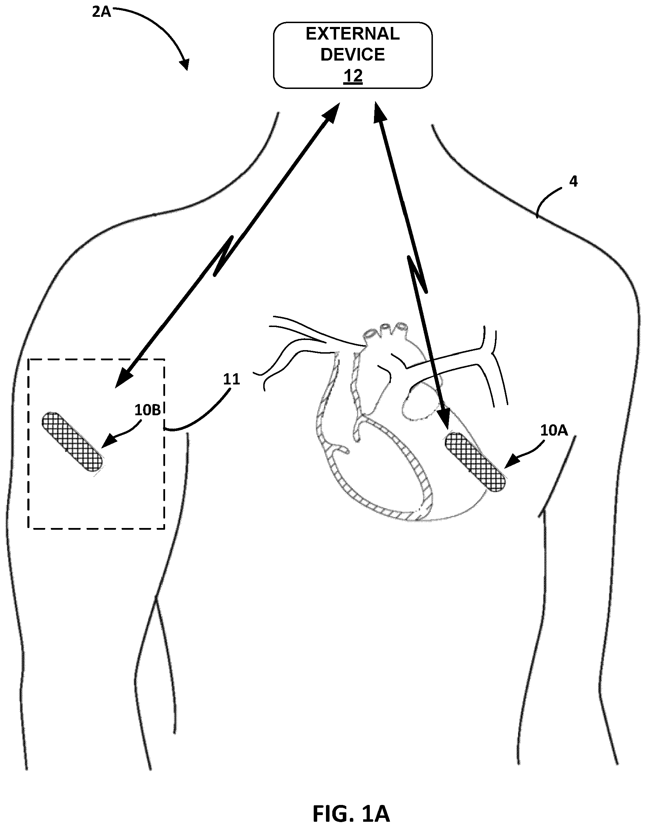

FIG. 1A is a conceptual diagram of an example medical system 2A in conjunction with a patient 4, in accordance with one or more techniques of this disclosure. Patient 4 ordinarily, but not necessarily, will be a human. For example, patient 4 may be an animal needing ongoing monitoring for cardiac conditions. System 2 includes medical device 10A and medical device 10B (collectively, "medical devices 10") . Each of medical devices 10 may include various sensors, e.g., electrodes, optical sensors, or needles, integrated with a housing of the medical device, or may be coupled to one or more leads that carry one or more sensors. System 2 may also include one or more external devices 12.

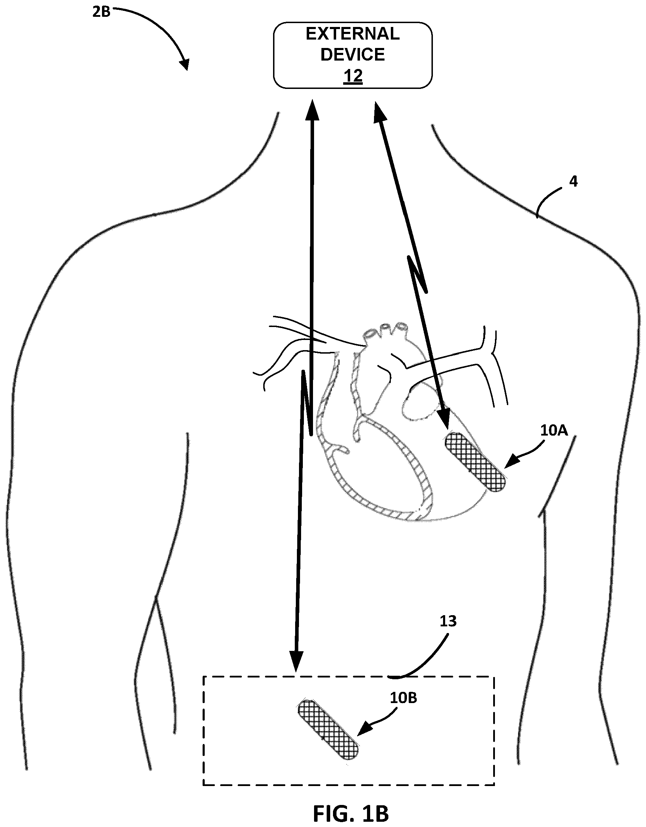

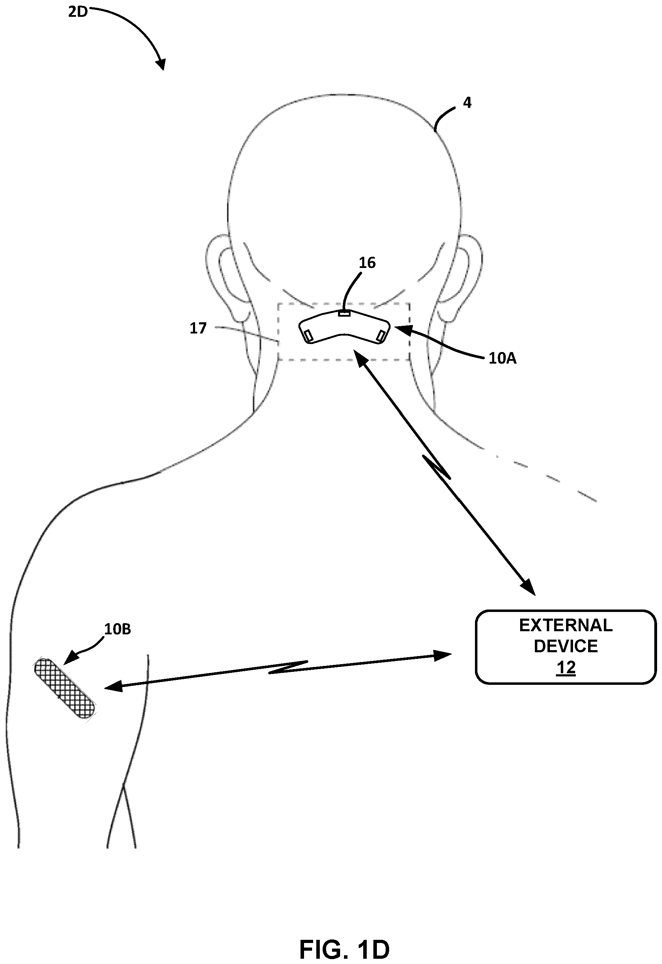

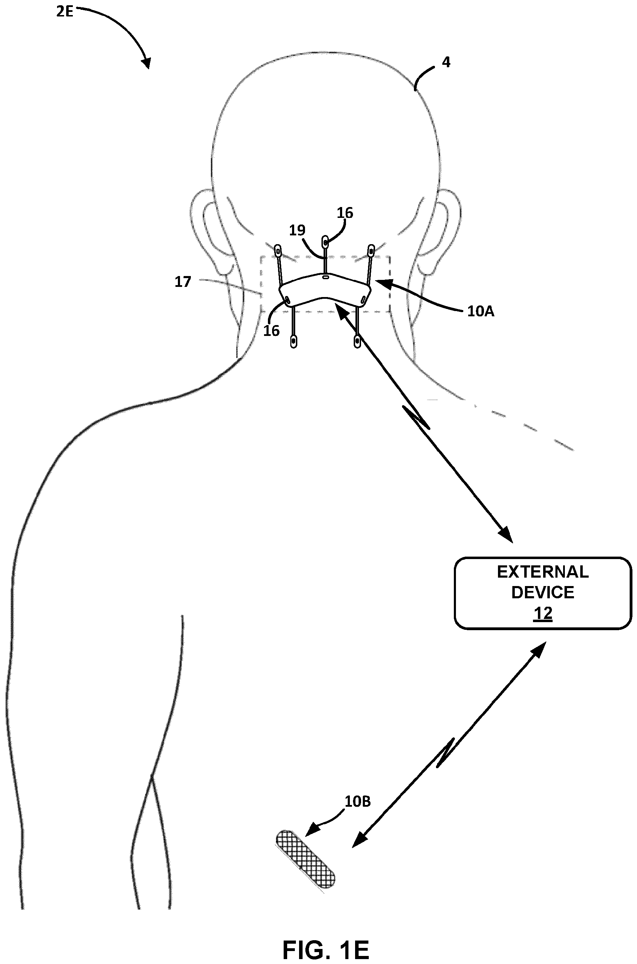

The example techniques may be used with medical devices 10, which may be configured to be in wireless communication with at least one of external computing device 12 and other devices not pictured in FIG. 1A. In some examples, one or both of medical devices 10 may be implanted within patient 4, e.g. subcutaneously. In some examples, one or both of medical devices 10 may be adhered to the skin or otherwise externally attached to or worn by patient 4. For example, medical device 10A may be implanted outside of a thoracic cavity of patient 4 (e.g., pectoral location illustrated in FIGS. 1A and 1B or cranial location illustrated in FIGS. 1D and 1E), and medical device 10B may be attached externally in target region 11, which is located on the upper arm of patient 4. In some examples, medical device 10A may be positioned near the sternum near or just below the level of the heart of patient 4, e.g., at least partially within the cardiac silhouette. In some examples, medical device 10B may be positioned in the region of the abdomen, thigh, leg, and shoulder of patient 4.

In some examples, medical device 10A may sense cardiac electrical signals (e.g., ECG signals) via a plurality of electrodes and/or operate as a therapy delivery device. For example, medical device 10A may operate as a therapy delivery device to deliver electrical signals to the heart of patient 4, such as an implantable pacemaker, a cardioverter, and/or defibrillator, a drug delivery device that delivers therapeutic substances to patient 4 via one or more catheters, or as a combination therapy device that delivers both electrical signals and therapeutic substances. In examples in which medical device 10A also operates as a pacemaker, a cardioverter, and/or defibrillator, or otherwise monitors the electrical activity of the heart, medical device 10A may sense electrical signals attendant to the depolarization and repolarization of the heart of patient 4 via electrodes coupled to at least one lead. In some examples, medical device 10A can provide pacing pulses to the heart of patient 4 based on the electrical signals sensed within the heart of patient 4. Medical device 10A may also provide defibrillation therapy and/or cardioversion therapy via electrodes located on at least one lead, as well as a housing electrode. Medical device 10A may detect arrhythmia of the heart of patient 4, such as fibrillation of ventricles, and deliver defibrillation therapy to the heart of patient 4 in the form of electrical pulses.

In some examples, medical device 10B may be configured to monitor blood glucose level and/or operate as a drug delivery device. For example, glucose levels monitored by medical device 10B may be used to control and adjust the administration of insulin to patient 4, e.g., by patient 4, a caregiver, medical device 10B, or another medical device. In some examples, either or both of medical device 10A and medical device 10B may be configured to monitor hydration level and/or hematocrit level of patient 4. For example, medical device 10A may be configured to determine hydration levels of patient 4 based on impedance measured via electrodes of medical device 10A, and either of the medical devices may include an optical sensor and be configured to determine hematocrit levels based on signals sensed using optical sensor. Such parameters may be indicative of a likelihood of a medical condition, such as stroke, in patient 4. In general, when a patient is dehydrated, their blood volume goes down and hematocrit goes up, which can lead to an increased tendency to clotting.

In some examples, medical device 10A is implantable and takes the form of the Reveal LINQ^{™} ICM, or another ICM similar to, e.g., a version or modification of, the LINQ^{™} ICM. For example, the medical devices depicted in FIG. 1D or FIG. 1E may be a version of modification of the LINQ^{™} ICM for cranial implantation, and medical devices may be configured for other anatomical implant locations in some examples. Such IMDs may facilitate relatively longer-term monitoring of patients during normal daily activities, and may periodically transmit collected data to a network service, such as the Medtronic CareLink^{®} Network. In some examples, medical device 10B takes the form of the Guardian^{™} Sensor 3, or another external or implantable glucose monitoring device. Although illustrated as separate devices in the example of FIG. 1A, in some examples a single implantable or external medical device may be configured for ECG, glucose level sensing, hydration level sensing, and/or hematocrit level sensing according to the techniques described herein.

External device 12 may be a computing device with a display viewable by a user and an interface for providing input to external device 12 (i.e., a user input mechanism). The user may be a physician technician, surgeon, electrophysiologist, clinician, or patient 4. In some examples, external device 12 may be a notebook computer, tablet computer, computer workstation, one or more servers, cellular phone, personal digital assistant, handheld computing device, networked computing device, or another computing device that may run an application that enables the computing device to interact with medical devices 10. For example, external device 12 may be a clinician, physician, or user programmer configured to communicate wirelessly with medical devices 10 and perform data transfers between external device 12 and medical devices 10. External device 12 is configured to communicate with medical devices 10 and, optionally, another computing device (not illustrated in FIG. 1A), via wired or wireless communication. External device 12, for example, may communicate via near-field communication (NFC) technologies (e.g., inductive coupling, NFC or other communication technologies operable at ranges less than 10-20 cm) and far-field communication technologies (e.g., Radio Frequency (RF) telemetry according to the 802.11 or Bluetooth^{®} specification sets, or other communication technologies operable at ranges greater than near-field communication technologies). In some examples, external device 12 may include a programming head that may be placed proximate to the body of patient 4 near the medical devices 10 in order to improve the quality or security of communication between medical devices 10 and external device 12.

In some examples, the user may use external device 12 to program or otherwise interface with medical devices 10. External device 12 may be used to program aspects of sensing or data analysis performed by medical devices 10 and/or therapies provided by medical devices 10. In addition, external device 12 may be used to retrieve data from medical devices 10. The retrieved data may include ECG data recorded by medical devices 10, e.g., due to a medical device 10 determining that one or more episodes of arrhythmia or another malady occurred during a time unit, or in response to a request to record the ECG data from patient 4 or another user. In some examples, the user may also use external device 12 to retrieve information from medical devices 10 regarding other sensed physiological parameters of patient 4, such as activity, hydration level, hematocrit level, glucose (and/or other blood analyte) concentration, a metric indicative of an atrial fibrillation condition, a metric indicative of a glucose condition (and/or other blood analyte condition), a metric indicative of hydration level, a metric indicative of hematocrit level, a health metric indicative of a risk of stroke, or posture. Additionally, one or more remote computing devices may interact with medical devices 10 in a manner similar to external device 12, e.g., to program medical devices 10 and/or retrieve data from medical devices 10, via a network.

Processing circuitries of medical devices 10 and/or external device 12 may be configured to perform the example techniques of this disclosure for determining a health metric based on atrial fibrillation and glucose levels, and in some cases hydration levels, hematocrit levels, or other blood analyte levels. For example, as described in greater detail elsewhere in this disclosure, the processing circuitries of system 2 may analyze electrical signals generated in response to the presence of glucose and/or other blood analyte(s) in the bloodstream, cardiac activity signals, and other signals generated by medical devices 10 to determine health metrics indicative of the risk of stroke of patient 4.

In some examples, medical device 10A may detect cardiac electrical signals (e.g., ECG signals) over a time unit via one or more electrodes. The ECG signals detected by medical device 10A may be filtered using one or more signal processing techniques (e.g., low-pass filter, high-pass filter, band-pass filter, band-stop filter, etc.). Processing circuitries of system 2, e.g., of medical device 10A, may analyze the ECG signals to detect atrial fibrillation episodes during a time unit, and determine a first metric indicative of an atrial fibrillation condition of patient 4 during the time unit.

In some examples, medical device 10B may detect electrical signals in response to the presence of glucose and/or other blood analyte(s) in the bloodstream during a time unit. The electrical signals detected by medical device 10B may be filtered using one or more signal processing techniques (e.g., low-pass filter, high-pass filter, band-pass filter, band-stop filter, etc.). Processing circuitries of system 2, e.g., of medical device 10B, may analyze the electrical signals to determine glucose levels during a time unit, and a second metric indicative of a glucose condition of patient 4 during the time unit.

In some examples, medical device 10A may detect electrical signals, e.g., impedance signals, in response to the change in hydration level during a time unit via the electrodes. The electrical signals detected by medical device 10A may be filtered using one or more signal processing techniques (e.g., low-pass filter, high-pass filter, band-pass filter, band-stop filter, etc.). Processing circuitries of system 2, e.g., of medical device 10A, may analyze the electrical signals to determine hydration levels during a time unit, and a third metric indicative of a hydration level of patient 4 during the time unit.

In some examples, medical device 10A or 10B may detect electrical signals, e.g., from an optical sensor, that indicate the amount of red blood cells in the blood of patient 4, which, like hydration level, reflects the circulating blood volume in patient, during a time unit. The electrical signals detected by medical device 10A or 10B may be filtered using one or more signal processing techniques (e.g., low-pass filter, high-pass filter, band-pass filter, band-stop filter, etc.). Processing circuitries of system 2, e.g., of medical device 10A or 10B, may analyze the electrical signals to determine hematocrit levels during a time unit, and a fourth metric indicative of a hematocrit condition of patient 4 during the time unit.

Depending on the first metric, the second metric, the third metric, and/or the fourth metric, the processing circuitries of system 2, e.g., of one of medical devices 10 or external device 2, may determine a health metric indicative of the risk of stroke of patient 4. In examples in which a medical device 10 determines the health metric, the medical device may further transmit the health metric to one or more external devices (e.g., external device 12) for review by a clinician and a possible medical intervention.

FIG. 1B is a conceptual diagram of an example medical system 2B in conjunction with a patient 4, in accordance with one or more techniques of this disclosure. Medical system 2B may be substantially similar to medical system 2A of FIG. 1A. However, medical device 10B of medical system 2B may be configured to be implanted in target region 13, which is located on the abdomen of patient 4. Medical device 10B implanted at target region 13 may be configured to detect blood glucose concentration or changes in blood glucose concentration, as well as other sensor signals described herein, in this area. In some examples, medical device 10B may be configured to detect the change with the circulating blood volume. In some examples, medical device 10B may need to employ different filters or other processing or signal conditioning techniques than those at target region 11 due to different types of noise at target region 13, such as abdominal muscle activity or other types of electrical activity. In other examples, medical device 10B may be configured to sense signals as described herein from other areas of patient 4 that may be outside of target regions 11 and 13.

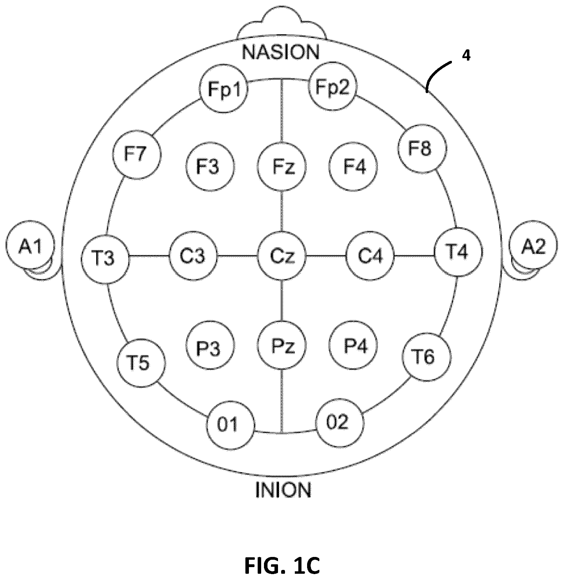

FIG. 1C is a diagram of the 10-20 map for electroencephalography (EEG) sensor measurements. As shown in FIG. 1C, various locations on the head of patient 4 may be targeted using the electrodes carried by medical device 10A. At the back of the head, medical device 10A may sense electrical signals at least one of P3, Pz or P4. At the side of the head, medical device 10A may sense electrical signals at least one of F7, T3, or T5 and/or at one or more of F8, T4, or T6. Medical device 10B is not shown in FIG. 1C. However, medical device 10A may be substantially similar to medical device 10A of FIGS. 1A-1B and may be in either of locations illustrated by FIGS. 1A-1B.

FIG. 1D is a conceptual diagram of an example medical system 2D in conjunction with a patient 4, in accordance with one or more techniques of this disclosure. Medical system 2D may be substantially similar to medical system 2A of FIG. 1A. However, medical device 10A of medical system 2D may be configured to be implanted in target region 17, which is located at a rear portion of the neck or the base of the skull of patient 4. In the illustrated example, medical device 10A of medical system 2D includes a housing that carries three electrodes 16 (one of which is labeled in FIG. 1D). Although three electrodes are shown for medical device 10A of medical system 2D, in other examples, two or four or more electrodes may be carried by the housing of medical device 10A of medical system 2D. As illustrated, the housing of medical device 10A can define a boomerang or chevronlike shape, which a central portion includes a vertex, with lateral portions extending laterally outward and from the central portion and also at a downward angle with respect to a horizontal axis of medical device 10A. In other examples, the housing of medical device 10A may be formed in other shapes, which may be determined by desired distances or angles between different electrodes carried by the housing. The configuration of the housing can facilitate placement either over the skin of patient 4 in a wearable or bandage-like form or for subcutaneous implantation. As such, a relatively thin housing can be advantageous. Additionally, the housing of medical device 10A can be flexible in some embodiments, so that the housing can at least partially bend to correspond to the anatomy of the neck of patient 4 (e.g., with left and right lateral portions of the housing of medical device 10A bending anteriorly relative to the central portion of the housing of medical device 10A).

Medical device 10B implanted on the upper arm of patient 4 may be configured to sense detect blood glucose concentration or changes in blood glucose concentration, as well as other sensor signals described herein, in this area. For example, medical device 10B may include one or more optical hematocrit sensor and may be configured to detect the change with the circulating blood volume. In other examples, medical device 10B may be configured to sense signals as described herein from other areas of patient 4 that may be outside of the upper arm of patient 4.

FIG. 1E is a conceptual diagram of an example medical system 2E in conjunction with a patient 4, in accordance with one or more techniques of this disclosure. Medical system 2A may be substantially similar to medical system 2D of FIG. 1D. However, as an alternative or in addition to electrodes 16 on its housing, medical device 10A of medical system 2E further include electrode extensions 19 (one of which is labeled in FIG. 1E) including electrodes 16. As illustrated in FIG. 1E, electrode extensions 19 of medical device 10A include paddles such that one or more electrodes 16 are distributed on the paddles. In some examples, electrode extensions 19 of medical device 10A include one or more ring electrodes. In some examples, electrode extensions 19 of medical device 10A may be connected to the housing of medical device 10A via header pins. In some examples, electrode extensions 19 of medical device 10A may be permanently attached to the housing of medical device 10A.

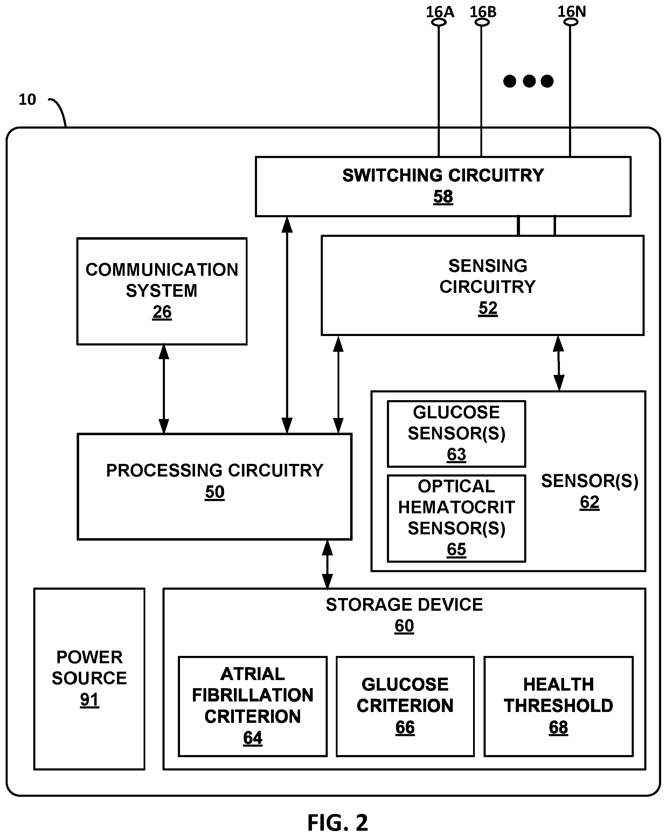

In the example of FIG. 2E, medical device 10B is implanted on the abdomen of patient 4 and may be configured to sense detect blood glucose concentration or changes in blood glucose concentration, as well as other sensor signals described herein, in this area. For example, medical device 10B may include one or more optical hematocrit sensor and may be configured to detect the change with the circulating blood volume. In other examples, medical device 10B may be configured to sense signals as described herein from other areas of patient 4 that may be outside of the abdomen of patient 4.

FIG. 2 is a functional block diagram illustrating an example configuration of a medical device 10 in accordance with one or more techniques described herein. In the illustrated example, a single medical device 10 is configured for ECG, glucose or other blood analyte level, hydration level, and hematocrit level sensing. However, in some examples, as discussed above with respect to FIG. 1A, different medical devices may be configured for respectively sensing one or more of these parameters, such as different devices being configured for ECG and glucose level sensing, respectively. In the illustrated example, medical device 10 includes electrodes 16A-16N (collectively, "electrodes 16"), communication system 26, processing circuitry 50, sensing circuitry 52, storage device 60, switching circuitry 58, sensor(s) 62, and power source 91.

Processing circuitry 50 may include fixed function circuitry and/or programmable processing circuitry. Processing circuitry 50 may include any one or more of a microprocessor, a controller, a digital signal processor (DSP), an application specific integrated circuit (ASIC), a field-programmable gate array (FPGA), or equivalent discrete or analog logic circuitry. In some examples, processing circuitry 50 may include multiple components, such as any combination of one or more microprocessors, one or more controllers, one or more DSPs, one or more ASICs, or one or more FPGAs, as well as other discrete or integrated logic circuitry. The functions attributed to processing circuitry 50 herein may be embodied as software, firmware, hardware, or any combination thereof.

In some examples, sensing circuitry 52 may be configured to monitor heart activities. For example, sensing circuitry 52 may sense ECG signals via electrodes 16, in order to facilitate monitoring activities of the heart. Sensing circuitry 52 may also be configured to measure impedance, e.g., for determining hydration levels, via electrodes 16. In some examples, sensing circuitry 52 may be selectively coupled to electrodes 16 via switching circuitry 58 (e.g., to select the electrodes 16 and polarity) for ECG and impedance sensing.

In some examples, sensing circuitry 52 may further monitor signals from sensor(s) 62, which may include one or more glucose sensor(s) 63, optical hematocrit sensor(s) 65, accelerometers, pressure sensors, and/or optical sensors, as examples. Any suitable glucose sensor(s) 63 may be used to detect blood glucose concentration or changes in blood glucose concentration. For example, glucose sensor(s) 63 may include electrodes or other elements exposed to fluid of patient 4, e.g., interstitial fluid, where a signal generated by the sensor varies based on the concentration of glucose in the fluid. Any suitable optical hematocrit sensor(s) 65 may be used to detect the change with the circulating blood volume.

In some examples, sensing circuitry 52 may include one or more filters and amplifiers for filtering and amplifying signals received from electrodes 16 and sensors 62. Sensing circuitry 52 may perform smoothing of the signals (e.g., by performing digital and/or analog filtering), such that processing circuitry 50 may perform various other techniques of this disclosure based on the smoothened signals.

Processing circuitry 50 may analyze the ECG signals received from sensing circuity 52 in order to detect atrial fibrillation episodes, and determine a first metric indicative of an atrial fibrillation condition of patient 4 during a time unit. Atrial fibrillation is a form of cardiac arrhythmia where the atria of the heart fail to continuously contract in synchrony with the ventricles of the heart. During atrial fibrillation, the atria undergo rapid and unorganized electrical depolarization, so that no contractile force is produced. As a result, blood may pool in the atria, increasing the risk of stroke. Additionally, medical studies have shown that glycemic control reduces the amount of atrial fibrillation for many patients.

Based on the ECG signals sensed via electrodes 16, processing circuitry 50 may make a determination of whether patient 4 experiences atrial fibrillation during a time unit. For example, processing circuitry 50 may determine whether or not the ECG signals satisfy an atrial fibrillation criterion 64 as stored in storage device 60. Based on the determination, processing circuitry 50 may determine a first metric indicative of an atrial fibrillation condition of patient 4 during the time unit.

In some examples, the first metric may include a first amount of time or a first percentage of the time unit that patient 4 experiences atrial fibrillation during the time unit. In other examples, the first metric may include a first amount of time or a first percentage of the time unit that patient 4 does not experience atrial fibrillation during the time unit.

Processing circuitry 50 may analyze the glucose signals received from sensing circuity 52 in order to determine a second metric indicative of a glucose condition (e.g., average glucose concentration) of patient 4 during the time unit. Elevated blood glucose concentration (e.g., hyperglycemia) can contribute to the buildup of clots and/or deposits inside vessels that supply blood to the neck and brain. As a result, elevated blood glucose may cause a narrowing of the blood vessel wall, increasing the risk of stroke. Based on the glucose signals sensed via glucose sensor(s) 63 and/ or electrodes 16, processing circuitry 50 may make a determination of whether the glucose concentration of patient 4 is outside of a target range. For example, processing circuitry 50 may determine whether or not the glucose signals satisfy a glucose criterion 66 as stored in storage device 60. Based on the determination, processing circuitry 50 may determine a second metric indicative of the glucose condition of patient 4 during the time unit.

In some examples, the second metric may include a second amount of time or a second percentage of the time unit that the glucose concentration (e.g., shorter term average of glucose concentration) of patient 4 is outside of a target range during the time unit. In other examples, the second metric may include a second amount of time or a second percentage of the time unit that the glucose concentration of patient 4 is outside of a target range during the time unit.

Once processing circuitry 50 determines a first metric indicative of an atrial fibrillation condition and a second metric indicative of a glucose condition of patient 4, processing circuitry 50 may determine a health metric of patient 4 based on the first metric and the second metric. For example, processing circuitry 50 may determine the health metric based on a multiplication of (and/or other mathematical operation(s), such as a sum, difference, average, weighted average, ratio, etc., performed on) the first metric and the second metric. In some examples, the health metric may be indicative of the risk of stroke of patient 4.

In some examples, processing circuitry 50 may analyze impedance via electrodes in order to determine a third metric indicative of hydration level (e.g., average hydration level) of patient 4 during the time unit. The electrical signals detected by medical device 10A may be filtered using one or more signal processing techniques (e.g., low-pass filter, high-pass filter, band-pass filter, band-stop filter, etc.). Dehydration can contribute to the buildup of clots and/or deposits inside vessels that supply blood to the neck and brain, increasing the risk of stroke. Processing circuitry 50 may further determine the health metric of patient 4 based on the third metric.

In some examples, processing circuitry 50 may analyze the hematocrit signals received from sensing circuity 52 in order to determine a fourth metric indicative of a hydration level (e.g., average hydration level) of patient 4 during the time unit. Processing circuitry 50 may further determine the health metric of patient 4 based on the fourth metric.

In some examples, processing circuitry 50 may generate an alert to patient 4 when a value of the health metric satisfies a health threshold 68 stored in storage device 60. The health threshold 68 may be a value, e.g., programmable, associated with a clinically significant risk of an adverse medical condition of patient 4, such as stroke. Some examples may utilize multiple health thresholds 68 associated with differing levels of risk and/or associated with a particular adverse medical condition such as stroke. In such examples, processing circuitry 50 may utilize different alerts and/or alerting mechanisms depending on which of the thresholds is satisfied. For example, processing circuitry 50 may send an alert to an external device to inform patient 4, which may enable patient 4 proactively to seek medical attention prior to receiving instructions for medical intervention. In this manner, patient 4 may be empowered to take action, as needed, to address his or her stroke status, which may help improve clinical outcomes for patient 4.

In further examples, processing circuitry 50 may generate a health metric trend based on values of the health metric of patient 4 over a period of time. The historical health graph may be viewed with various display methodologies such as line, area, bar, point, etc., at user's selection.

In some examples, processing circuitry 50 may control the timing of measurements of physiological parameters based on a schedule. For example, processing circuitry 50 may control the measurement of physiological parameters on a periodic basis, such as on an hourly or daily basis. In one example, sensing circuitry 52, via sensor(s) 62 and/or electrodes 16, may measure physiological parameters during a particular portion of a day. As an example, sensing circuitry 52 may measure physiological parameters every twenty minutes for a predetermined number of hours, such as between 8 am and 5 pm. In some examples, sensing circuitry 52 may be configured to sample physiological parameters at a particular sampling rate. In such examples, sensing circuitry 52 may be configured to perform downsampling in order to decrease the throughput rate for processing circuitry 50. This may be particularly advantageous where sensing circuitry 52 configured at a high sampling rate when active.

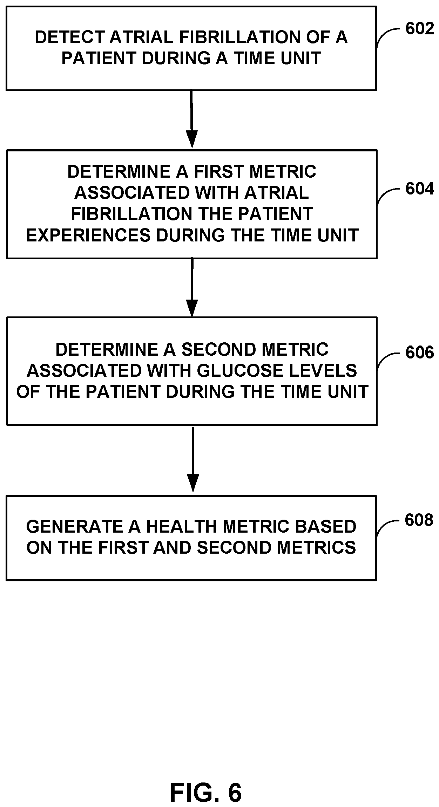

In the example illustrated in FIG. 2, processing circuitry 50 is capable of performing the various techniques described with reference to FIGS. 6-8. In various examples, processing circuitry 50 may perform one, all, or any combination of the plurality of techniques discussed in greater detail below. In some examples, processing circuitry 50 may store the glucose concentration, metric indicative of an atrial fibrillation condition, metric indicative of a glucose condition, metric indicative of a hydration level (or blood volume), and health metric indicative of a risk of stroke to storage device 60.

Communication system 26 may include any suitable hardware, firmware, software or any combination thereof for communicating with another device, such as external device 12, another networked computing device, or another medical device or sensor. Under the control of processing circuitry 50, communication system 26 may receive downlink telemetry from, as well as send uplink telemetry to external device 12 or another device with the aid of an internal or external antenna. In addition, processing circuitry 50 may communicate with a networked computing device via an external device (e.g., external device 12) and a computer network, such as the Medtronic CareLink^{®} Network. Communication system 26 may be configured to transmit and/or receive signals via inductive coupling, electromagnetic coupling, near-field communications, RF communication, Bluetooth^{®}, WI-FI^{™}, or other proprietary or non-proprietary wireless communication schemes. For example, processing circuitry 50 may provide data to be uplinked to external device 12 via communication system 26 and control signals using an address/data bus. In some examples, communication system 26 may provide received data to processing circuitry 50 via a multiplexer.

In some examples, storage device 60 includes computer-readable instructions that, when executed by processing circuitry 50, cause medical device 10 and processing circuitry 50 to perform various functions attributed to medical device 10 and processing circuitry 50 herein. Storage device 60 may include any volatile, non-volatile, magnetic, optical, or electrical media. For example, storage device 60 may include random access memory (RAM), read-only memory (ROM), non-volatile RAM (NVRAM), electrically-erasable programmable ROM (EEPROM), erasable programmable ROM (EPROM), flash memory, or any other digital media. Storage device 60 may store, as examples, programmed values for one or more operational parameters of medical device 10 and/or data collected by medical device 10 for transmission to another device using communication system 26. Data stored by storage device 60 and transmitted by communication system 26 to one or more other devices may include cardiac electrical or mechanical data, glucose, hydration level, and/or other blood analyte(s) concentration, metric indicative of an atrial fibrillation condition, metric indicative of a glucose condition, metric indicative of a hydration level, and/or health metric, which may be indicative of a risk of stroke.

The various components of medical device 10 are coupled to power source 91, which may include a rechargeable or non-rechargeable battery. A non-rechargeable battery may be capable of holding a charge for several years, while a rechargeable battery may be inductively charged from another device, such as external device 12, on a daily, weekly, or annual basis, for example.

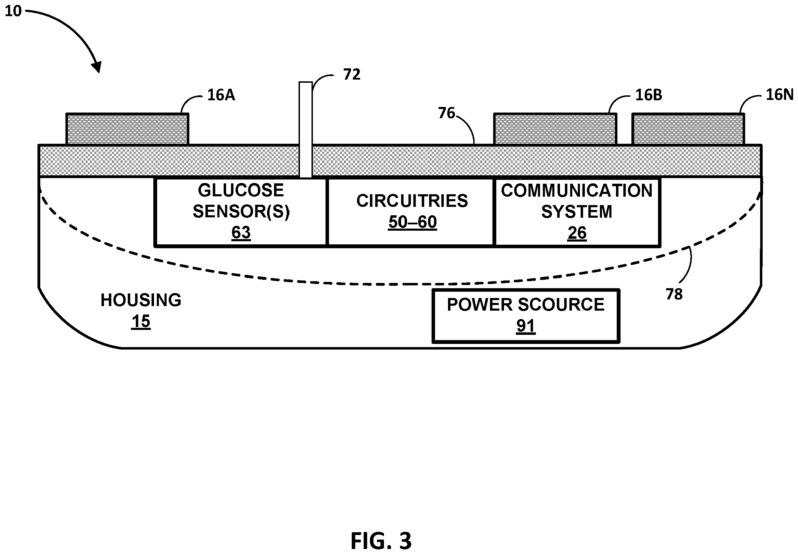

FIG. 3 is a conceptual side-view diagram illustrating an example configuration of a medical device 10. In the illustrated example, a single medical device 10 is configured for ECG, glucose level sensing, and/or hydration level sensing. However, in some examples, as discussed above with respect to FIG. 1A, different medical devices may be configured for ECG, glucose level sensing, and/or hydration level sensing, respectively.

In the example shown in FIG. 3, medical device 10 may include a medical device having a housing 15 and an insulative cover 76. Electrodes 16 may be formed or placed on an outer surface of cover 76. Communication system 26, circuitries 50-60 and/or sensor(s) 62, described above with respect to FIG. 2, may be formed or placed on an inner surface of cover 76, or otherwise within housing 15. Sensor(s) 62 may include one or more glucose sensor(s) 63 and one or more optical hematocrit sensor(s) 65 located within housing 15. Glucose sensor(s) 63 may be coupled to or include one or more sensing elements 72 that extends through cover 76, and is thereby exposed to fluid of patient 4. Each of the one or more sensing elements 72 may include one or more electrodes, or is configured, e.g., as a needle or the like with a lumen, to allow fluid to come into contact with sensor(s) 63 within housing 15. In some examples, insulative cover 76 may be positioned over housing 15, such that housing 15 and insulative cover 76 enclose communication system 26, glucose sensor(s) 63, other sensor(s) 62, and/or circuitries 50-60, and protect them from fluids.

One or more of communication system 26, sensor(s) 62, glucose sensor(s) 63, optical hematocrit sensor(s) 65, and/or circuitries 50-60 may be formed on the inner side of insulative cover 76, such as by using flip-chip technology. Insulative cover 76 may be flipped onto a housing 15. When flipped and placed onto housing 15, the components of medical device 10 formed on the inner side of insulative cover 76 may be positioned in a gap 78 defined by housing 15. Power source 91 of medical device 10 may be housed within housing 15. Electrodes 16 may be electrically connected to switching circuitry 58 through one or more vias (not shown) formed through insulative cover 76. Insulative cover 76 may be formed of sapphire (i.e., corundum), glass, perylene, and/or any other suitable insulating material. Housing 15 may be formed from titanium or any other suitable material (e.g., a biocompatible material). Electrodes 16 may be formed from any of stainless steel, titanium, platinum, iridium, or alloys thereof. In addition, electrodes 16 may be coated with a material such as titanium nitride or fractal titanium nitride, although other suitable materials and coatings for such electrodes may be used.

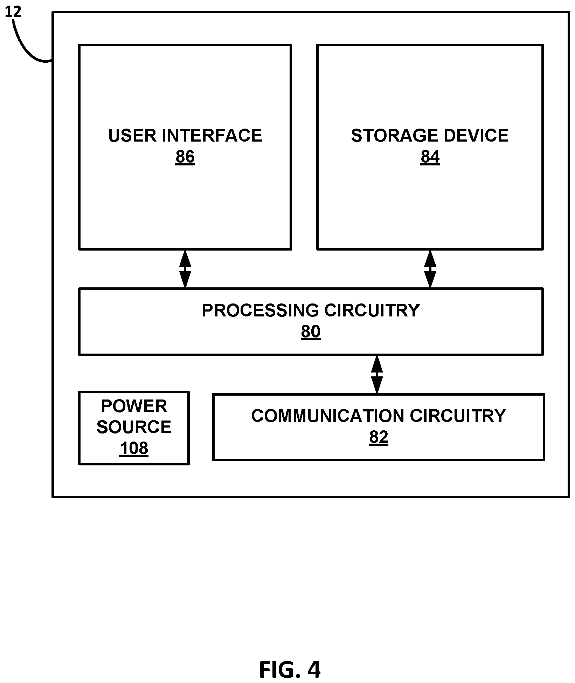

FIG. 4 is a block diagram illustrating an example configuration of components of external device 12. In the example of FIG. 4, external device 12 includes processing circuitry 80, communication circuitry 82, storage device 84, and user interface 86.

Processing circuitry 80 may include one or more processors that are configured to implement functionality and/or process instructions for execution within external device 12. For example, processing circuitry 80 may be capable of processing instructions stored in storage device 84. Processing circuitry 80 may include, for example, microprocessors, DSPs, ASICs, FPGAs, or equivalent discrete or integrated logic circuitry, or a combination of any of the foregoing devices or circuitry. Accordingly, processing circuitry 80 may include any suitable structure, whether in hardware, software, firmware, or any combination thereof, to perform the functions ascribed herein to processing circuitry 80.

Communication circuitry 82 may include any suitable hardware, firmware, software, or any combination thereof for communicating with other devices, such as medical devices 10. Under the control of processing circuitry 80, communication circuitry 82 may receive downlink telemetry from, as well as send uplink telemetry to, medical devices 10, or another device. Communication circuitry 82 may be configured to transmit or receive signals via inductive coupling, electromagnetic coupling, Near Field Communication (NFC), RF communication, Bluetooth^{®}, WI-FI^{™}, or other proprietary or non-proprietary wireless communication schemes. Communication circuitry 82 may also be configured to communicate with devices other than medical devices 10 via any of a variety of forms of wired and/or wireless communication and/or network protocols.

Storage device 84 may be configured to store information within external device 12 during operation. Storage device 84 may include a computer-readable storage medium or computer-readable storage device. In some examples, storage device 84 includes one or more of a short-term memory or a long-term memory. Storage device 84 may include, for example, RAM, DRAM, SRAM, magnetic discs, optical discs, flash memories, or forms of EPROM or EEPROM. In some examples, storage device 84 is used to store data indicative of instructions for execution by processing circuitry 80. Storage device 84 may be used by software or applications running on external device 12 to temporarily store information during program execution. Storage device 84 may also store historical health metric data, current health metric data, etc.

External device 12 can additionally or alternatively include a peripheral pointing device, such as a mouse, via which the user may interact with the user interface. In some examples, a display of external device 12 may include a touch screen display, and a user may interact with external device 12 via the display. It should be noted that the user may also interact with external device 12 remotely via a networked computing device.

Data exchanged between external device 12 and medical devices 10 may include operational parameters (e.g., such as a communication rate). External device 12 may transmit data including computer readable instructions, which, when implemented by medical devices 10, may control medical devices 10 to change one or more operational parameters and/or export collected data. For example, processing circuitry 80 may transmit an instruction to medical devices 10, which requests medical devices 10 to export collected data (e.g., ECG data, AF episode data, AF condition metrics, glucose levels, glucose condition metrics, and/or health metrics) to external device 12. In turn, external device 12 may receive the collected data from medical devices 10 and store the collected data in storage device 84.

In some examples, processing circuitry 80 of external device 12 may perform some or all of the techniques described herein for determining values of a health metric based on information received from one or more medical devices 10. For example, processing circuitry 80 may receive AF condition metric values, glucose condition metric values, and/or hydration level metric values from one or more medical devices 10, or may determine such metric values from glucose level data and AF episode data received from one or more medical devices 10. Processing circuitry 80 may determine values of the health metric based on the AF condition metric values, glucose condition metric values, and/or hydration level metric values as described herein.

A user, such as a clinician or patient 4, may interact with external device 12 through user interface 86. User interface 86 includes a display (not shown), such as a liquid crystal display (LCD) or a light emitting diode (LED) display or other type of screen, with which processing circuitry 80 may present information related to medical devices 10, e.g., ECG data, AF episode data, glucose concentration data, hydration level data, and/or historical health metric data. In addition, user interface 86 may include an input mechanism to receive input from the user. The input mechanisms may include, for example, any one or more of buttons, a keypad (e.g., an alphanumeric keypad), a peripheral pointing device, a touch screen, or another input mechanism that allows the user to navigate through user interfaces presented by processing circuitry 80 of external device 12 and provide input. In other examples, user interface 86 also includes audio circuitry for providing audible notifications, instructions, or other sounds to the user, receiving voice commands from the user, or both.

Power source 108 delivers operating power to the components of external device 12. Power source 108 may include a battery and a power generation circuit to produce the operating power. In some embodiments, the battery may be rechargeable to allow extended operation. Recharging may be accomplished by electrically coupling power source 108 to a cradle or plug that is connected to an alternating current (AC) outlet. In addition or alternatively, recharging may be accomplished through proximal inductive interaction between an external charger and an inductive charging coil within external device 12. In other embodiments, traditional batteries (e.g., nickel cadmium or lithium ion batteries) may be used. In addition, external device 12 may be directly coupled to an alternating current outlet to power external device 12. Power source 108 may include circuitry to monitor power remaining within a battery. In this manner, user interface 86 may provide a current battery level indicator or low battery level indicator when the battery needs to be replaced or recharged. In some cases, power source 108 may be capable of estimating the remaining time of operation using the current battery.

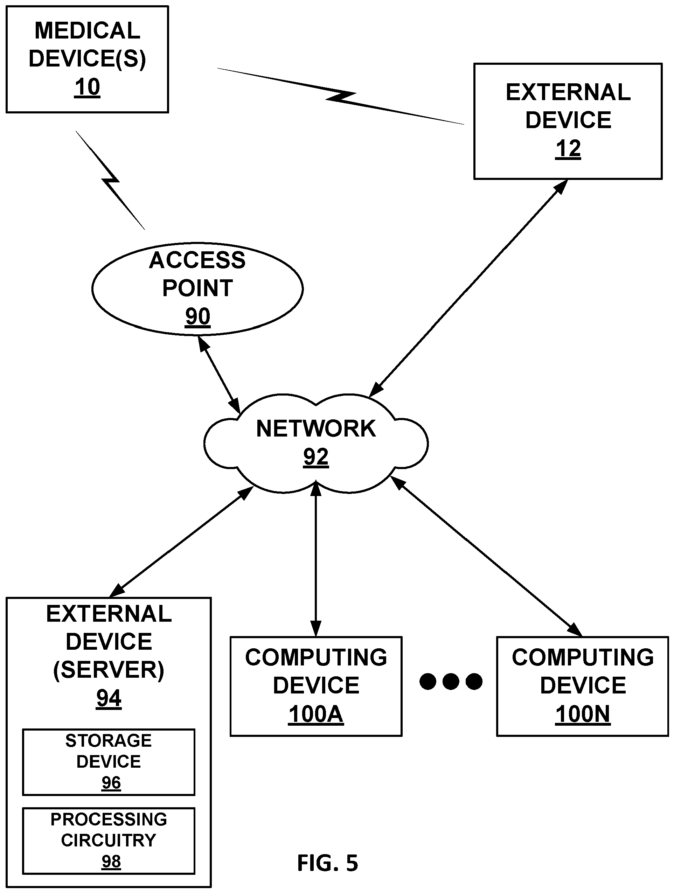

FIG. 5 is a block diagram illustrating an example system that includes an access point 90, a network 92, external computing devices, such as a server 94, and one or more other computing devices 100A-100N (collectively, "computing devices 100"), which may be coupled to medical devices 10 and external device 12 via network 92, in accordance with one or more techniques described herein. In this example, medical devices 10 may use communication system 26 to communicate with external device 12 via a first wireless connection, and to communicate with an access point 90 via a second wireless connection. In the example of FIG. 5, access point 90, external device 12, server 94, and computing devices 100 are interconnected and may communicate with each other through network 92. Network 92 may comprise a local area network, wide area network, or global network, such as the Internet. The system of FIG. 5 may be implemented, for some aspects, with general network technology and functionality similar to that provided by the Medtronic CareLink^{®} Network.

Access point 90 may include a device that connects to network 92 via any of a variety of connections, such as telephone dial-up, digital subscriber line (DSL), or cable modem connections. In other examples, access point 90 may be coupled to network 92 through different forms of connections, including wired or wireless connections. In some examples, access point 90 may be a user device, such as a tablet or smartphone, that may be co-located with the patient. Medical devices 10 may be configured to transmit data, such as ECG data, AF episode data, AF condition metric values, glucose concentration data, glucose condition metric values, hydration level data, and/or health metric values, to access point 90. Access point 90 may then communicate the retrieved data to server 94 via network 92.

In some cases, server 94 may be configured to provide a secure storage site for data that has been collected from medical devices 10 and/or external device 12. In some cases, server 94 may assemble data in web pages or other documents for viewing by trained professionals, such as clinicians, via computing devices 100. One or more aspects of the illustrated system of FIG. 5 may be implemented with general network technology and functionality, which may be similar to that provided by the Medtronic CareLink^{®} Network.

In some examples, one or more of computing devices 100 may be a tablet or other smart device located with a clinician, by which the clinician may program, receive data from, and/or interrogate medical devices 10. For example, the clinician may access data collected by medical devices 10 through a computing device 100, such as when patient 4 is in between clinician visits, to check on the status of a medical condition. In some examples, the clinician may enter instructions for medical intervention for patient 4 into an application executed by computing device 100, such as based on patient data known to the clinician. Device 100 then may transmit the instructions for medical intervention to another of computing devices 100 located with patient 4 or a caregiver of patient 4. For example, such instructions for medical intervention may include an instruction to change a drug dosage, timing, or selection, to schedule a visit with the clinician, or to seek medical attention. In further examples, a computing device 100 may generate an alert to patient 4 based on the status of a medical condition of patient 4, which may enable patient 4 proactively to seek medical attention prior to receiving instructions for a medical intervention. In this manner, patient 4 may be empowered to take action, as needed, to address his or her medical status, which may help improve clinical outcomes for patient 4. In some examples, the severity of alerts or actions requested may vary based on satisfaction of different thresholds by health metric values, as described above.

In the example illustrated by FIG. 5, server 94 includes a storage device 96, e.g., to store data retrieved from medical devices 10, and processing circuitry 98. Although not illustrated in FIG. 5, computing devices 100 may similarly include a storage device and processing circuitry. Processing circuitry 98 may include one or more processors that are configured to implement functionality and/or process instructions for execution within server 94. For example, processing circuitry 98 may be capable of processing instructions stored in storage device 96 (e.g., stored in memory). Processing circuitry 98 may include, for example, microprocessors, DSPs, ASICs, FPGAs, or equivalent discrete or integrated logic circuitry, or a combination of any of the foregoing devices or circuitry. Accordingly, processing circuitry 98 may include any suitable structure, whether in hardware, software, firmware, or any combination thereof, to perform the functions ascribed herein to processing circuitry 98.

Storage device 96 may include a computer-readable storage medium or computer-readable storage device. In some examples, storage device 96 includes one or more of a short-term memory or a long-term memory. Storage device 96 may include, for example, RAM, DRAM, SRAM, magnetic discs, optical discs, flash memories, or forms of EPROM or EEPROM. In some examples, storage device 96 is used to store data indicative of instructions for execution by processing circuitry 98.

In some examples, processing circuitry 98 of server 94, may perform some or all of the techniques described herein for determining values of a health metric based on information received from one or more medical devices 10. For example, processing circuitry 98 may receive AF condition metric values, glucose condition metric values, and/or hydration level metric values from one or more medical devices 10, or may determine such metric values from glucose level data, AF episode data, and/or hydration level data received from one or more medical devices 10. Processing circuitry 80 may determine values of the health metric based on the AF, glucose condition, and/or hydration level metric values as described herein.

FIG. 6 is a flow diagram illustrating an example operation for generating a health metric indicative of the risk of stroke of patient 4 based on signals sensed via one or more medical devices 10, such as medical device 10A or medical device 10B, in accordance with one or more techniques of this disclosure. The example operation of FIG. 6 is described as being performed by processing circuitry, which may include processing circuitry 50 of one or more medical devices 10, processing circuitry 80 of external device 12, and/or processing circuitry 98 of server 94.

In some examples, the processing circuitry, e.g., processing circuitry 50 of medical device 10A, may receive a first signal indicating cardiac activity of patient 4 during a time unit. For example, processing circuitry 50 may obtain ECG data from sensing circuitry 52 during a pre-defined time unit. Processing circuitry, e.g., processing circuitry 50 of medical device 10A, may detect atrial fibrillation episodes of patient 4 (and/or other cardiac activity of patient 4) during the time unit based on the obtained ECG data (602).

In some examples, processing circuitry, e.g., processing circuitry 50 of medical device 10A or processing circuitry 98 of server 94, may determine a first metric associated with atrial fibrillation (and/or other cardiac activity of patient 4) the patient experiences during the time unit (604). In some examples, the first metric may include a first amount of time or a first percentage of the time unit that patient 4 experiences atrial fibrillation during the time unit. In other examples, the first metric may include a first amount of time or a first percentage of the time unit that patient 4 does not experience atrial fibrillation during the time unit.

Processing circuitry, e.g., processing circuitry 50 of medical device 10B, may further receive a second signal indicating or associated with a glucose condition of patient 4 during the time unit. For example, processing circuitry 50 of medical device 10B may obtain glucose concentration data from one or more glucose sensors 63 during the time unit, and processing circuitry 50 of medical device 10B and/or processing circuitry 98 of server 94 may make a determination of whether the glucose concentration levels of patient 4 are outside of a target range. In implementing the technologies, devices, systems, methods, etc. disclosed herein, a glucose condition or glucose level of a patient may be determined by measurement of glucose concentration in body fluid of patient 4, and/or by measurement of the concentration in body fluid of patient 4 of other analyte(s) that are related to or associated with glucose concentration, such as hemoglobin, hemoglobin A1c, albumin, insulin, proinsulin, etc. Where one or more such analytes are measured instead of or in addition to glucose, glucose sensors 63 can be appropriately configured to sense the desired analyte(s), to facilitate determination of glucose level(s) or condition(s) based on such analyte(s).

In some examples, processing circuitry, e.g., processing circuitry 50 of medical device 10B and/or processing circuitry 98 of server 94, may determine a second metric indicative of or associated with the glucose condition of patient 4 during the time unit (606). In some examples, the second metric may include a second amount of time or a second percentage of the time unit that the glucose concentration of patient 4 is outside of a target range during the time unit. In other examples, the second metric may include a second amount of time or a second percentage of the time unit that the glucose concentration of patient 4 is outside of a target range during the time unit.

Depending on both the first metric and second metric, processing circuitry, e.g., processing circuitry 50 of a medical device 10 or processing circuitry 98 of server 94, may determine a health metric indicative of the risk of stroke of patient 4 (608). In some examples, the processing circuitry may determine the health metric based on a multiplication of (and/or other mathematical operation(s), such as a sum, difference, average, weighted average, ratio, etc., performed on) the first metric and the second metric.

Using glucose concentration or atrial fibrillation condition alone to calculate the health risk of an adverse patient conditions, such as the risk of stroke, may be prone to false triggering. The techniques of this disclosure may improve the detection capabilities of a medical system including medical devices 10. Using both the first metric indicative of an atrial fibrillation condition and the second metric indicative of or associated with a glucose condition of patient 4 to determine a health metric, e.g., indicative of the risk of stroke, of patient 4 may be more robust than using only the glucose concentration or the atrial fibrillation condition alone.

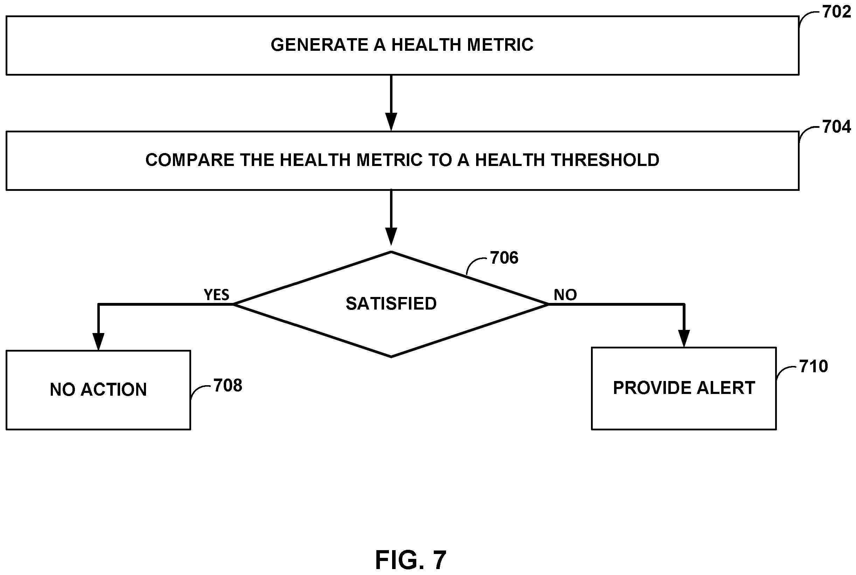

FIG. 7 is a flow diagram illustrating an example operation for generating an alert based on a health metric determined by one or more medical devices, such as medical device 10A or medical device 10B, in accordance with one or more techniques of this disclosure. The example operation of FIG. 7 is described as being performed by processing circuitry, which may include processing circuitry 50 of one or more medical devices 10, processing circuitry 80 of external device 12, and/or processing circuitry 98 of server 94.

In some examples, processing circuitry, e.g., processing circuitry 50 of medical device 10A or processing circuitry 98 of server 94, may determine a health metric indicative of the risk of stroke based on, e.g., a first metric indicative of an atrial fibrillation condition and a second metric indicative of or associated with a glucose condition of patient 4 (702).

In some examples, the processing circuitry, e.g., processing circuitry 50 of a medical device 10 or processing circuitry 98 of server 94, may further determine whether or not the health metric satisfies health threshold 68 as stored in storage device 60 (704). The processing circuitry may make this determination in any of various ways. In some examples, the processing circuitry may determine that the health metric satisfies health threshold 68 based on the health metric meeting a predefined threshold value.

In response to determining that the health metric satisfies health threshold 68 as stored in storage device 60 ("YES" branch of 704), processing circuitry, e.g., processing circuitry 50 of a medical device 10 or processing circuitry 98 of server 94, may determine a health metric for the next time unit (706). However, if the processing circuitry determines that the health metric has not satisfied health threshold 68 as stored in storage device 60 ("NO" branch of 704), the processing circuitry may send an alert to an external device (e.g., external device 12) to inform patient 4 or a clinician that the patient may need assistance or therapeutic intervention.

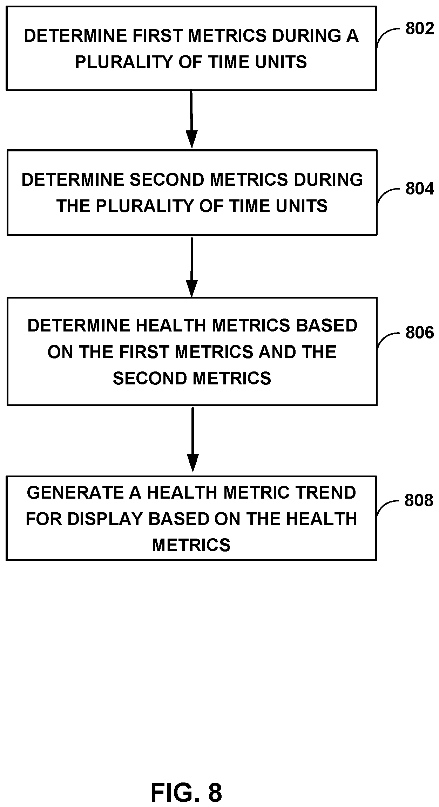

FIG. 8 is a flow diagram illustrating an example operation for generating a health metric trend for display, in accordance with one or more techniques of this disclosure. The example operation of FIG. 8 is described as being performed by processing circuitry, which may include processing circuitry 50 of one or more medical devices 10, processing circuitry 80 of external device 12, and/or processing circuitry 98 of server 94.

In some examples, processing circuitry, e.g., processing circuitry 50 of medical device 10A may obtain ECG data from sensing circuitry 52 during a plurality of time units. In some examples, processing circuitry 50 may obtain ECG data during a predefined time unit (e.g., one minute, five minutes, etc.) from sensing circuitry 52 every twenty minutes, hourly over a period of time. For example, processing circuitry, e.g., processing circuitry 50 of medical device 10A may obtain ECG data at a sampling rate of twice every hour over the course of a 24-hour time period. In another example, processing circuitry, e.g., processing circuitry 50 of medical device 10A may obtain ECG data at a sampling rate of once every hour during specific times of the day, such as between 8:00 am and 5:00 pm. In some examples, processing circuitry, e.g., processing circuitry 50 of medical device 10A may control sensing circuitry 52 to perform random measurements at random times during a set time period (e.g., randomly throughout each day). Based on the received ECG data, processing circuitry 50 may determine a plurality of first metrics indicative of atrial fibrillation during the plurality of time units (802).

In some examples, processing circuitry, e.g., processing circuitry 50 of medical device 10B may obtain glucose data from sensing circuitry 52 and/or glucose sensor(s) 63 during the plurality of time units. Based on the received glucose data, processing circuitry, e.g., processing circuitry 50 of medical device 10B may determine a plurality of second metrics indicative of glucose concentration of patient 4 during the plurality of time units (804).

Depending on both the first metrics and second metrics, processing circuitry, e.g., processing circuitry 50 of a medical device 10 or processing circuitry 98 of server 94 may determine health metrics indicative of the risk of stroke of patient 4 during the plurality of time units (806).

In some examples, processing circuitry, e.g., processing circuitry 50 of a medical device 10 or processing circuitry 98 of server 94 may further generate a health metric trend for display based on the health metrics (808). The health metric trend may be viewed with various display methodologies such as line, area, bar, point, etc., at user's selection.

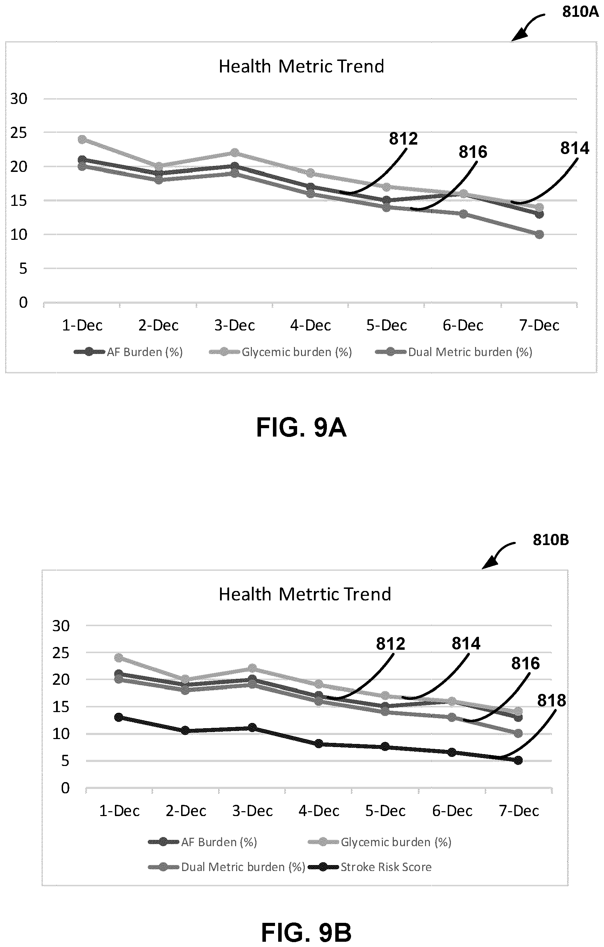

FIG. 9A is a graph illustrating an example historical health metric trend 810A generated according to the techniques of this disclosure. Historical health metric trend 810A represents an exemplary presentation of data outputted by medical device 10, external device 12, and/or server 94. However, it should be understood that other computer devices may be configured to output or present such data.

In some examples, historical health metric trend 810A includes a historical AF burden trend 812, a historical glycemic burden trend 814, and a historical dual metric burden trend 816. Historical AF burden trend 812 shows a trend of values, e.g., daily, of the percentage of time, e.g., during the day, that patient 4 is in atrial fibrillation. Glycemic burden trend 814 shows a trend of values, e.g., daily, of the percentage of time the glucose level of patient 4 is outside of a target range. Dual metric burden trend 816 shows a trend of values, e.g., daily, of the percentage of time when patient 4 experiences atrial fibrillation and the glucose level is outside of the target range.

FIG. 9B is a graph illustrating an example historical health metric 810B generated according to the techniques of this disclosure. Historical health metric trend with risk score 810B represents an exemplary presentation of data outputted by medical device 10, external device 12, and/or server 94. However, it should be understood that other computer devices may be configured to output or present such data.

In some examples, historical health metric trend 810B includes a historical AF burden trend 812, a historical glycemic burden trend 814, a historical dual metric burden trend 816, and a historical stroke score trend 818. In some examples, a stroke risk score may be generated based on health metrics. For example, a stroke risk score may be calculated based on AF burden and glycemic burden. As shown in FIG. 8C, historical stroke score trend 818 shows the risk of stroke of patient 4 reduces over time as AF burden trend 812, glycemic burden trend 814, and dual metric burden trend 816 decreased over time.

The techniques described in this disclosure may be implemented, at least in part, in hardware, software, firmware, or any combination thereof. For example, various aspects of the techniques may be implemented within one or more microprocessors, DSPs, ASICs, FPGAs, or any other equivalent integrated or discrete logic QRS circuitry, as well as any combinations of such components, embodied in external devices, such as physician or patient programmers, stimulators, or other devices. The terms "processor" and "processing circuitry" may generally refer to any of the foregoing logic circuitry, alone or in combination with other logic circuitry, or any other equivalent circuitry, and alone or in combination with other digital or analog circuitry.

For aspects implemented in software, at least some of the functionality ascribed to the systems and devices described in this disclosure may be embodied as instructions on a computer-readable storage medium such as RAM, ROM, NVRAM, DRAM, SRAM, Flash memory, magnetic discs, optical discs, flash memories, or forms of EPROM or EEPROM. The instructions may be executed to support one or more aspects of the functionality described in this disclosure.

In addition, for some aspects, the functionality described herein may be provided within dedicated hardware and/or software modules. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components. Also, the techniques could be fully implemented in one or more circuits or logic elements. The techniques of this disclosure may be implemented in a wide variety of devices or apparatuses, including an medical device, an external programmer, a combination of a medical device and external programmer, an integrated circuit (IC) or a set of ICs, and/or discrete electrical circuitry, residing in a medical device and/or external programmer.

Furthermore, although described primarily with reference to examples that provide an infection status to indicate a device pocket infection in response to detecting temperature changes in the device pocket, other examples may additionally or alternatively automatically modify a therapy in response to detecting the infection status in the patient. The therapy may be, as examples, a substance delivered by an implantable pump, a delivery of antibiotics, etc.

## Claims

1. A system (10) comprising:
electrocardiogram sensing circuitry configured to sense an electrocardiogram of a patient;
glucose sensing circuitry configured to sense glucose levels of the patient; and processing circuitry (50) configured to:
detect atrial fibrillation of the patient during a time unit based on the electrocardiogram of the patient,
determine a first metric, wherein the first metric is associated with atrial fibrillation the patient experiences during the time unit, and wherein the first metric comprises a first amount of time that the patient experiences atrial fibrillation during the time unit,
determine a second metric, wherein the second metric is associated with glucose levels of the patient during the time unit, and wherein the second metric comprises a second amount of time that the glucose level of the patient is outside of a target range during the time unit,
generate a health metric comprising a stroke metric, wherein the stroke metric indicates a probability that the patient has experienced or will experience a stroke, and wherein the health metric is determined based on the first and second metrics, and
output an alert based on the health metric, and/or transmit the health metric to an external device (12) for review by a clinician.

2. The system of claim 1, wherein the health metric is determined based on a multiplication of the first metric and the second metric.

3. The system of claim 1, wherein the system further comprises sensing circuitry (52) configured to sense hydration levels of the patient, wherein the processing circuitry is further configured to determine a third metric, wherein the third metric is associated with hydration levels of the patient during the time unit, and the health metric is further determined based on the third metric and preferably, wherein, to sense the hydration levels, the sensing circuitry is configured to sense at least one of impedance or optical hematocrit levels.

4. The system of claim 1, wherein the processing circuitry is configured to:
compare the health metric to a health threshold (68); and
output the alert in response to the health metric satisfying the health threshold or wherein the processing circuitry is configured to:
determine a plurality of first metrics during a plurality of time units,
determine a plurality of second metrics during the plurality of time units,
determine a plurality of health metrics based on the plurality of first metrics and the plurality of second metrics, and
generate a health metric trend (810A) for display based on the plurality of health metrics.

5. The system of claim 1, wherein the glucose sensing circuitry is configured to measure a concentration of glucose in a body fluid of the patient.

6. The system of claim 1, wherein the glucose sensing circuitry is configured to measure a concentration of an analyte in a body fluid of the patient, wherein the analyte is associated with a glucose concentration or wherein the analyte comprises at least one of hemoglobin, hemoglobin A1c, albumin, insulin, or proinsulin.

7. The system of claim 1, wherein the processing circuitry comprises processing circuitry of an external computing device (12) in wireless communication with one or more other devices comprising the electrocardiogram sensing circuitry and the glucose sensing circuitry.

8. The system of claim 1, further comprises a housing (15) carrying the plurality of electrodes and containing the electrocardiogram sensing circuitry and the processing circuitry, and/or
wherein the housing is configured to be implanted within the patient, and/or
wherein the housing is configured to be implanted subcutaneously, or wherein the housing is configured to be disposed on an external surface of skin of the patient, or wherein the housing contains the glucose monitoring circuitry.

9. The system of claim 8, further comprising a first implantable medical device (10B) comprising the electrocardiogram sensing circuitry and an external medical device (10A) comprising the glucose sensing circuitry.

## Patentansprüche

1. System (10), umfassend:
Elektrokardiogrammerfassungsschaltung, die konfiguriert ist, um ein Elektrokardiogramm eines Patienten zu erfassen;
eine Glucoseerfassungsschaltung, die konfiguriert ist, um Glucosespiegel des Patienten zu erfassen; und
eine Verarbeitungsschaltung (50), die konfiguriert ist zum:
Erkennen eines Vorhofflimmerns des Patienten während einer Zeiteinheit basierend auf dem Elektrokardiogramm des Patienten,
Bestimmen einer ersten Metrik, wobei die erste Metrik dem Vorhofflimmern zugeordnet ist, das der Patient während der Zeiteinheit erfährt, und wobei die erste Metrik eine erste Zeitdauer umfasst, die der Patient das Vorhofflimmern während der Zeiteinheit erfährt,
Bestimmen einer zweiten Metrik, wobei die zweite Metrik Glucosespiegeln des Patienten während der Zeiteinheit zugeordnet ist, und wobei die zweite Metrik eine zweite Zeitdauer umfasst, die der Glucosespiegel des Patienten während der Zeiteinheit außerhalb eines Zielbereichs liegt,
Erzeugen einer Gesundheitsmetrik, umfassend eine Schlaganfallmetrik, wobei die Schlaganfallmetrik eine Wahrscheinlichkeit angibt, dass der Patient einen Schlaganfall erfahren hat oder erfahren wird, und wobei die Gesundheitsmetrik basierend auf der ersten und der zweiten Metrik bestimmt wird, und
Ausgeben einer Warnung basierend auf der Gesundheitsmetrik, und/oder Übertragen der Gesundheitsmetrik an eine externe Vorrichtung (12) für eine Überprüfung durch einen Kliniker.

2. System nach Anspruch 1, wobei die Gesundheitsmetrik basierend auf einer Multiplikation der ersten Metrik und der zweiten Metrik bestimmt wird.

3. System nach Anspruch 1, wobei das System ferner eine Erfassungsschaltung (52) umfasst, die konfiguriert ist, um Hydratationsspiegel des Patienten zu erfassen, wobei die Verarbeitungsschaltung ferner konfiguriert ist, um eine dritte Metrik zu bestimmen, wobei die dritte Metrik Hydratationsspiegeln des Patienten während der Zeiteinheit zugeordnet ist, und die Gesundheitsmetrik ferner basierend auf der dritten Metrik bestimmt wird und vorzugsweise, wobei, um die Hydrationsspiegel zu erfassen, die Erfassungsschaltung konfiguriert ist, um mindestens einen von Impedanzspiegeln oder Spiegeln eines optischen Hämatokrits zu erfassen.

4. System nach Anspruch 1, wobei die Verarbeitungsschaltung konfiguriert ist zum:
Vergleichen der Gesundheitsmetrik mit einer Gesundheitsschwelle (68); und
Ausgeben der Warnung als Reaktion darauf, dass die Gesundheitsmetrik die Gesundheitsschwelle erfüllt, oder wobei die Verarbeitungsschaltung konfiguriert ist zum:
Bestimmen einer Vielzahl von ersten Metriken während einer Vielzahl von Zeiteinheiten,
Bestimmen einer Vielzahl von zweiten Metriken während der Vielzahl von Zeiteinheiten,
Bestimmen einer Vielzahl von Gesundheitsmetriken basierend auf der Vielzahl von ersten Metriken und der Vielzahl von zweiten Metriken, und
Erzeugen eines Gesundheitsmetriktrends (810A) für eine Anzeige basierend auf der Vielzahl von Gesundheitsmetriken.

5. System nach Anspruch 1, wobei die Glucoseerfassungsschaltung konfiguriert ist, um eine Glucosekonzentration in einem Körperfluid des Patienten zu messen.

6. System nach Anspruch 1, wobei die Glucoseerfassungsschaltung konfiguriert ist, um eine Konzentration eines Analyten in einem Körperfluid des Patienten zu messen, wobei der Analyt einer Glucosekonzentration zugeordnet ist oder wobei der Analyt mindestens eines von Hämoglobin, Hämoglobin A1c, Albumin, Insulin oder Proinsulin umfasst.

7. System nach Anspruch 1, wobei die Verarbeitungsschaltung eine Verarbeitungsschaltung einer externen Rechenvorrichtung (12) in drahtloser Kommunikation mit einer oder mehreren anderen Vorrichtungen umfasst, umfassend die Elektrokardiogrammerfassungsschaltung und die Glucoseerfassungsschaltung.

8. System nach Anspruch 1, das ferner ein Gehäuse (15) umfasst, das die Vielzahl von Elektroden trägt und die Elektrokardiogrammerfassungsschaltung und die Verarbeitungsschaltung enthält, und/oder
wobei das Gehäuse konfiguriert ist, um innerhalb des Patienten implantiert zu werden, und/oder
wobei das Gehäuse konfiguriert ist, um subkutan implantiert zu werden, oder wobei das Gehäuse konfiguriert ist, um auf einer externen Oberfläche einer Haut des Patienten angeordnet zu werden, oder wobei das Gehäuse die Glucoseüberwachungsschaltung enthält.

9. System nach Anspruch 8, ferner umfassend eine erste implantierbare medizinische Vorrichtung (10B), umfassend die Elektrokardiogrammerfassungsschaltung und eine externe medizinische Vorrichtung (10A), umfassend die Glucoseerfassungsschaltung.

## Revendications

1. Système (10) comprenant :
un circuit de détection d'électrocardiogramme configuré pour détecter un électrocardiogramme d'un patient ;
un circuit de détection de glucose configuré pour détecter des niveaux de glucose du patient ; et
un circuit de traitement (50) configuré pour :
détecter une fibrillation auriculaire du patient pendant une unité de temps sur la base de l'électrocardiogramme du patient,
déterminer une première métrique, dans lequel la première métrique est associée à une fibrillation auriculaire subie par le patient pendant l'unité de temps, et dans lequel la première métrique comprend une première quantité de temps pendant laquelle le patient subit une fibrillation auriculaire pendant l'unité de temps,
déterminer une deuxième métrique, dans lequel la deuxième métrique est associée à des niveaux de glucose du patient pendant l'unité de temps, et dans lequel la deuxième métrique comprend une seconde quantité de temps pendant laquelle le niveau de glucose du patient est hors d'une plage cible pendant l'unité de temps,
générer une métrique de santé comprenant une métrique d'accident vasculaire cérébral, dans lequel la métrique d'accident vasculaire cérébral indique une probabilité que le patient ait subi ou subira un accident vasculaire cérébral, et dans lequel la métrique de santé est déterminée sur la base de la première et de la deuxième métrique, et
émettre une alerte sur la base de la métrique de santé, et/ou transmettre la métrique de santé à un dispositif externe (12) pour examen par un clinicien.

2. Système selon la revendication 1, dans lequel la métrique de santé est déterminée sur la base d'une multiplication de la première métrique et de la deuxième métrique.

3. Système selon la revendication 1, dans lequel le système comprend en outre un circuit de détection (52) configuré pour détecter des niveaux d'hydratation du patient, dans lequel le circuit de traitement est en outre configuré pour déterminer une troisième métrique, dans lequel la troisième métrique est associée à des niveaux d'hydratation du patient pendant l'unité de temps, et la métrique de santé est en outre déterminée sur la base de la troisième métrique et de préférence, dans lequel, pour détecter des niveaux d'hydratation, le circuit de détection est configuré pour détecter au moins l'un parmi un niveau d'impédance ou un niveau d'hématocrite optique.

4. Système selon la revendication 1, dans lequel le circuit de traitement est configuré pour :
comparer la métrique de santé à un seuil de santé (68) ; et
émettre l'alerte en réponse au fait que la métrique de santé satisfait le seuil de santé ou dans lequel le circuit de traitement est configuré pour :
déterminer une pluralité de premières métriques pendant une pluralité d'unités de temps,
déterminer une pluralité de deuxièmes métriques pendant la pluralité d'unités de temps,
déterminer une pluralité de métriques de santé sur la base de la pluralité de premières métriques et de la pluralité de deuxièmes métriques, et
générer une tendance de métrique de santé (810A) pour affichage sur la base de la pluralité de métriques de santé.

5. Système selon la revendication 1, dans lequel le circuit de détection de glucose est configuré pour mesurer une concentration de glucose dans un fluide corporel du patient.

6. Système selon la revendication 1, dans lequel le circuit de détection de glucose est configuré pour mesurer une concentration d'un analyte dans un fluide corporel du patient, dans lequel l'analyte est associé à une concentration de glucose ou dans lequel l'analyte comprend au moins l'un parmi l'hémoglobine, l'hémoglobine Ale, l'albumine, l'insuline ou la proinsuline.

7. Système selon la revendication 1, dans lequel le circuit de traitement comprend un circuit de traitement d'un dispositif informatique externe (12) en communication sans fil avec un ou plusieurs autres dispositifs comprenant le circuit de détection d'électrocardiogramme et le circuit de détection de glucose.

8. Système selon la revendication 1, comprenant en outre un boîtier (15) portant la pluralité d'électrodes et contenant le circuit de détection d'électrocardiogramme et le circuit de traitement, et/ou
dans lequel le boîtier est conçu pour être implanté à l'intérieur du patient, et/ou
dans lequel le boîtier est conçu pour être implanté par voie sous-cutanée, ou dans lequel le boîtier est conçu pour être disposé sur une surface externe de la peau du patient, ou dans lequel le boîtier contient le circuit de surveillance de glucose.

9. Système selon la revendication 8, comprenant en outre un premier dispositif médical implantable (10B) comprenant le circuit de détection d'électrocardiogramme et un dispositif médical externe (10A) comprenant le circuit de détection de glucose.
